# EUROPÄISCHE PATENTANMELDUNG

(11) **EP 1 929 938 A1**
(43) Veröffentlichungstag der Anmeldung: **11.06.2008**
(21) Anmeldenummer: 06125337.3
(22) Anmeldetag: 04.12.2006
(51) Int. Cl.: A61B 5/00, G01N 27/36, G01N 27/403, G01N 33/00

(54) **Vorrichtung zur Messung des Partialdrucks von Kohlendioxid**

(71) Anmelder: SENTEC AG, 4106 Therwil (CH)
(72) Erfinder: Kleinleugenmors, Adrianus, 4144, Hofstetten (CH)
(74) Vertreter: Dr. Graf & Partner

(57) **Zusammenfassung**

Die Vorrichtung (2) zur Messung des Partialdrucks von Kohlendioxid, umfassend ein Gehäuse (3) mit einer pH-Glaselektrode (4) sowie einer Referenzelektrode (6), wobei die Referenzelektrode (6) ein mit einem Diaphragma (6d) verschlossenes Schaftglas (6b) umfasst, wobei die pH-Glaselektrode (4) und die Referenzelektrode (6) einen Innenelektrolyt (4c, 6c) aufweisen, wobei die pH-Glaselektrode (4) und die Referenzelektrode (6) Elektrodenkontaktierungen (4a, 6a) aufweisen, die das Potential der Elektroden einer Messvorrichtung (13) zuführen, und wobei die Elektrodenkontaktierung (6a) im Innern der Referenzelektrode (6) aus einem mit Silberchlorid beschichteten Silberdraht besteht.

## Beschreibung

Die Erfindung betrifft eine Vorrichtung zur Messung des Partialdrucks von Kohlendioxid gemäss dem Oberbegriff von Anspruch 1.

### Stand der Technik

Es ist bekannt den CO₂-Gehalt des Bluts im menschlichen oder tierischen Körper zu bestimmen, indem mit einem Sensor an der Hautoberfläche der Partialdruck von Kohlendioxid gemessen wird. Grundlegende Arbeiten zu diesem Sensortyp wurden von J.W. Severinghaus geleistet, weshalb ein derartiger Sensor auch als "transkutane CO₂-Sensor nach Severinghaus" bezeichnet wird. Dieser Sensortyp ist beispielsweise im folgenden Artikel im Detail beschrieben: "The current Status of Transcutaneous Blood Gas Analysis and Monitoring", J.W. Severinghaus, BLOOD GAS NEWS, 1998, Vol. 7, No. 2.

Ein besonderer Vorteil des transkutaten CO₂-Sensors nach Severinghaus ist darin zu sehen, dass die beim Ausatmen auftretenden CO₂-Schwankungen ausgemittelt werden, und dass die Messung wenig störanfällig ist, weshalb dieser transkutane CO₂-Sensor beispielsweise häufig zur Überwachung von Patienten in Spitälern verwendet wird, um den CO₂-Gehält im Blut kontinuierlich zu überwachen.

Der transkutane CO₂-Sensor nach Severinghaus misst die Leitfähigkeitsänderung von Wasser, die sich beim Lösen von Kohlendioxid ergibt. Entsprechend der Gleichung

H₂O + CO₂ ↔ HCO_{3⁻} + H⁺ (Gleichung 1)

entstehen beim Lösen von CO₂ in Wasser Ionen, die eine Leitfähigkeitsänderung und eine pH-Änderung des Wassers bewirken. Die Leitfähigkeitsänderung korreliert mit dem jeweiligen Partialdruck von CO₂, so dass die Leitfähigkeitsänderung als Messgrösse für den vorliegenden CO₂-Partialdruck dient. Die Hydrolyse von Kohlendioxid in wässriger Lösung führt, entsprechend der Henderson-Hasselbalch'schen Gleichung

pH = pK + log[HCO_{3⁻} / [CO₂] (Gleichung 2)

zu einer partialdruckabhängigen pH-Änderung des Elektrolyten.

Der transkutane CO₂-Partialdruck wird auch als tcpCO₂ bezeichnet.

### Darstellung der Erfindung

Es hat sich gezeigt, dass transkutane CO₂-Sensoren nach Severinghaus ein instabiles Verhalten beziehungsweise ein Driftverhalten aufweisen, was den gemessenen tcpCO₂ -Wert und natürlich auch den daraus abgeleiteten arteriellen CO₂-Wert verfälscht. Beispielsweise hat sich gezeigt, dass transkutane CO₂-Sensoren nach Severinghaus innerhalb eines Zeitintervalls von beispielsweise 8 Stunden ein erhebliches Driftverhalten aufweisen.

Es ist daher Aufgabe der vorliegenden Erfindung eine Vorrichtung zur Messung des Partialdrucks von Kohlendioxid zu bilden, welche ein genaueres Messen des Kohlendioxidpartialdrucks erlaubt. Es ist insbesondere Aufgabe der vorliegenden Erfindung einen verbesserten Sensor zur Messung des CO₂-Gehaltes im Blut des menschlichen oder tierischen Körpers vorzuschlagen.

Diese Aufgabe wird gelöst mit einer Vorrichtung zur Messung des Partialdrucks von Kohlendioxid aufweisend die Merkmale von Anspruch 1. Die Unteransprüche 2 bis 5 betreffen weitere, vorteilhafte Ausgestaltungen.

Die Aufgabe wird insbesondere gelöst mit einer Vorrichtung zur Messung des Partialdrucks von Kohlendioxid, umfassend ein Gehäuse mit einer pH-Glaselektrode sowie einer Referenzelektrode, wobei die Referenzelektrode ein mit einem Diaphragma verschlossenes Schaftglas umfasst, wobei die pH-Glaselektrode und die Referenzelektrode einen Innenelektrolyt aufweisen, wobei die pH-Glaselektrode und die Referenzelektrode Elektrodenkontaktierungen aufweisen, die das Potential der Elektroden einer Messvorrichtung zuführen, und wobei die Elektrodenkontaktierung im Innern der Referenzelektrode und vorzugsweise auch die Elektrodenkontaktierung der pH-Glaselektrode aus einem mit Silberchlorid beschichteten Silberdraht besteht.

Die erfindungsgemässe Vorrichtung zur Messung des Partialdrucks von Kohlendioxid weist den Vorteil auf, dass diese bezüglich störender Einflüsse wesentlich stabiler ist. Wird diese Vorrichtung zur Messung des transkutaten CO₂-Gehaltes verwendet, so ist damit auch ein langfristig genaues Messen des tcpCO₂ -Wertes und des daraus abgeleiteten CO₂-Gehaltes im Blut möglich.

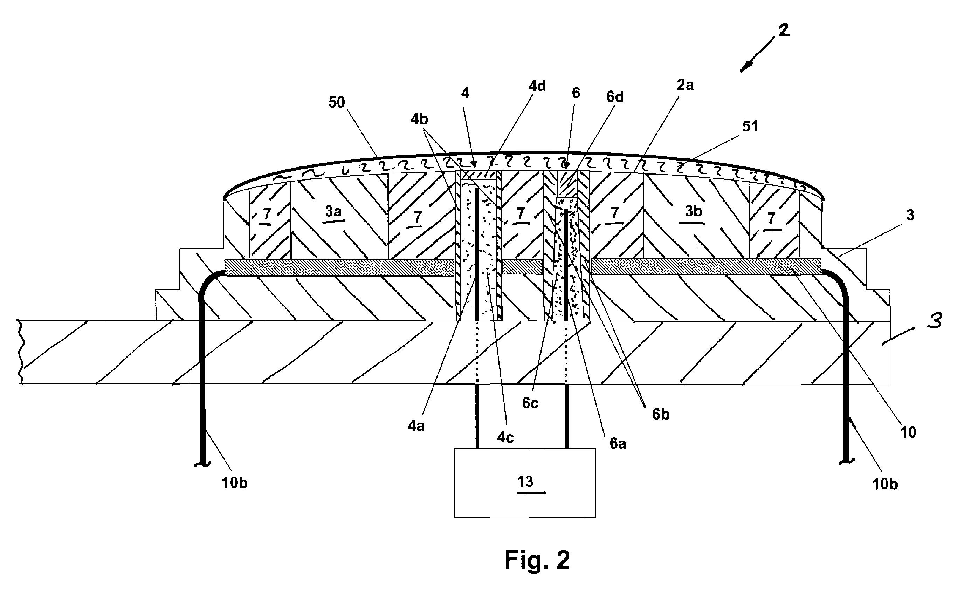

Die erfindungsgemässe Vorrichtung verzichtet auf die bei einem transkutanen CO₂-Sensor nach Severinghaus übliche Ag/AgCl-Referenzelektrode, welche an der Stirnseite des Sensors angeordnet ist, und üblicherweise als Silberblock ausgestaltet ist. Die erfindungsgemässe Vorrichtung verwendet als Referenzelektrode eine mit einem Diaphragma versehene Elektrode aus Glas, wobei sich im Inneren der Referenzelektrode ein mit Silberchlorid beschichteter Silberdraht befindet. Diese Anordnung bewirkt, wie in der nachfolgenden Figur 2 im Detail beschrieben, eine wesentliche Verbesserung des Driftverhaltens.

### Kurze Beschreibung der Zeichnungen

Die zur Erläuterung der Ausführungsbeispiele verwendeten Zeichnungen zeigen:
- Fig. 1: einen Längsschnitt durch einen bekannten transkutanen CO₂-Sensor;
- Fig. 2: einen Längsschnitt durch ein Ausführungsbeispiel des erfindungsgemässen transkutanen CO₂-Sensors.
Grundsätzlich sind in den Zeichnungen gleiche Teile mit gleichen Bezugszeichen versehen.

### Wege zur Ausführung der Erfindung

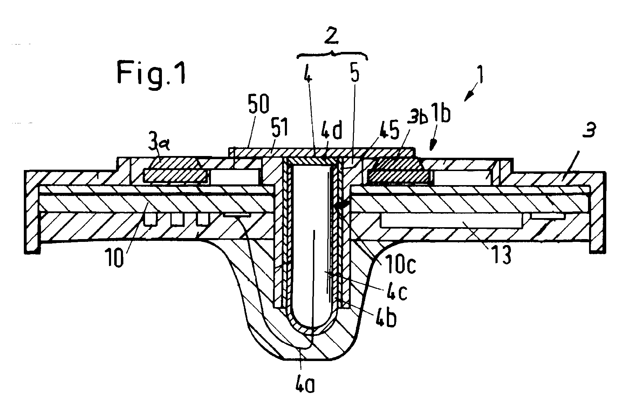

Der aus der Druckschrift WO 02/41770 bekannte, in Figur 1 dargestellte Sensor 1 erlaubt eine kombinierte Messung der arteriellen Sauerstoffsättigung SpO₂ und des transkutanen Kohlendioxidpartialdrucks tcpCO₂. Zur Messung der Sauerstoffsättigung weist der Sensor 1 ein Pulsoximetrie-Messystem auf, welches eine zweifarbige Leuchtdiode 3a sowie einen Fotodetektor 3b umfasst. Der Sensor 1 umfasst zudem einen transkutanen CO₂-Sensor 2 nach Severinghaus zur Messung des transkutanen Kohlendioxidpartialdrucks tcpCO₂. Diese Vorrichtung 2 zur Messung des Partialdrucks von Kohlendioxid umfasst, wie für einen transkutanen CO₂-Sensor nach Severinghaus üblich, eine Mikro-pH-Elektrode 4 sowie eine Ag/AgCl-Referenzelektrode 5, wobei diese Referenzelektrode 5 als Silberblock ausgestaltet ist, welcher die Mikro-pH-Elektrode 4 umgibt, und welche zusammen mit der Mikro-pH-Elektrode 4 eine gegen die Elektrolytlösung 51 hin ausgerichtete Stirnfläche 1b bildet. Der transkutane Kohlendioxidpartialdruck wird potentiometrisch gemessen, indem der pH-Wert der Elektrolytlösung 51 gemessen wird, welche über die gut gasdurchlässige, hydrophobe Membran 50 mit der Haut in Verbindung steht. Eine Änderung des CO₂-Wertes an der Hautoberfläche bewirkt, wie mit Gleichung 1 und 2 dargelegt, eine pH-Änderung der Elektrolytlösung 51. Der pH-Wert wird ermittelt indem das Potential zwischen der Mikro-pH-Elektrode 4 und der Ag/AgCl-Referenzelektrode 5 gemessen wird. Die Mikro-pH-Elektrode 4 ist über den elektrischen Innenableiter 4a Signal leitend mit einer Signalmessvorrichtung 13 verbunden.

Im Zentrum einer kreisförmigen Leiterplatte 10 ist eine als rundes Loch ausgestaltete Durchbrechung 10c ausgespart, durch welche die Vorrichtung 2 zur Messung des Partialdrucks von Kohlendioxid, umfassend die Mikro-pH-Elektrode 4 sowie die Ag/AgCl- Bezugselektrode 5, verläuft. Die Mikro-pH-Elektrode 4 ist als Glaselektrode ausgestaltet, und weist einen Innenelektrolyt 4c auf, welcher von einem Schaftglas 4b sowie einem Membranglas 4d umgeben ist. Zwischen der Mikro-pH-Elektrode 4 und der Referenzelektrode 5 ist ein Isolator 45 angeordnet.

Die Vorrichtung 2 zur Messung des Partialdrucks von Kohlendioxid umfasst somit eine pH-Elektrode 4, welche die Messelektrode bildet, sowie eine Silberhülse, einen Silberring oder einen Silberblock, welche die Referenzelektrode 5 bildet. Das Silber ist mit Silberchlorid beschichtet, und die Elektrolytlösung 51 mit einer bekannten Menge Chlorid versehen. Bisher wurde angenommen, dass die Referenzelektrode 5 ein stabiles Verhalten aufweist und somit ein stabiles Referenzsystem bildet. Es hat sich jedoch gezeigt, dass zwischen dem Silberchlorid und dem gemessenen CO₂ eine störende Reaktion auftritt.

Das CO₂ befindet sich in der Elektrolytlösung 51 in einem Gleichgewichtzustand mit Bikarbonat (HCO₃-), wie mit Gleichung 1 dargelegt.

Da in der Elektrolytlösung 51 Bikarbonat (HCO₃-) vorhanden ist, bildet sich mit Wasser eine kleine Menge Karbonat (CO₃²⁻) gemäss folgender Gleichung:

HCO_{3⁻} + H₂O ↔ CO₃^{2⁻} + H₃O⁺ (Gleichung 3)

Das auf der Oberfläche der Referenzelektrode vorhandene Silberchlorid reagiert mit dem Karbonat gemäss der Gleichung

CO₃^{2⁻} + 2 AgCl ↔ Ag₂CO₃ + 2 Cl⁻ (Gleichung 4)

insbesondere da Silberkarbonat unlöslicher ist als Silberchlorid. Das Potential der Ag/AgCl-Referenzelektrode 5 bleibt daher nicht konstant, weshalb eine Änderung der Potentialdifferenz zwischen pH-Elektrode 4 und Referenzelektrode 5 nicht ausschliesslich einer Änderung des Kohlendioxid-Partialdruckes zuzuschreiben ist. Der mit Gleichung 4 dargelegte Zusammenhang stört somit die Stabilität der Referenzelektrode 5, was zu einer Verfälschung der CO₂-Messung führt. Diese Zerstörung des Referenzsystems beziehungsweise des Referenzpotentials wird mit höherer CO₂ Konzentration zudem noch grösser.

Das Silberkarbonat ist zudem eine sehr lichtempfindliche Substanz, was bei Lichteinfall zur Folge hat, dass das Silberkarbonat wieder zerfällt, was wiederum eine Verschiebung des Referenzpotentials bewirkt und somit einen instabilen Messwert verursacht.

Da die Gleichung (4) ein Gleichgewicht darstellt, ist die Bildung von Silberkarbonat abhängig von der CO₂ Konzentration beziehungsweise vom CO₂ Partialdruck. Wird der CO₂ Partialdruck erhöht, wird Silberkarbonat gebildet. Wird daraufhin der CO₂ Partialdruck zu einem tieferen Wert reduziert, so verschiebt sich das Gleichgewicht in die Richtung von Silberchlorid, was bedeutet, dass Silberkarbonat in Silberchlorid umgewandelt wird. In beiden Fällen, das heisst Erhöhung oder Reduzierung der CO₂ Konzentration beziehungsweise des CO₂ Partialdrucks, ist das Potential des Referenzsystems nicht stabil und konstant, was zur Folge hat, dass die CO₂ Konzentration beziehungsweise der CO₂ Partialdruck nicht stabil gemessen werden kann.

Das Problem des sich verschiebenden Potentials des Referenzsystems wurde erfindungsgemäss dadurch gelöst, dass in der Vorrichtung 2 zur Messung des Partialdrucks von Kohlendioxid eine mit einem Diaphragma versehene Referenzelektrode verwendet wird.

Figur 2 zeigt in einem Längsschnitt ein Ausführungsbeispiel einer erfindungsgemässen Vorrichtung 2 zur Messung des Partialdrucks von Kohlendioxid. Die Vorrichtung 2 umfassend, angeordnet in einem Gehäuse 3 und in Kunststoff 7, eine miniaturisierte pH-Glaselektrode 4 mit Innenableiter 4a, Schaftglas 4b, Innenelektrolyt 4c und Membranglas 4d sowie eine separate Referenzelektrode 6 mit Innenableiter 6a, Schaftglas 6b, Innenelektrolyt 6c und Diaphragma 6d. Die Stirnseiten beider Elektroden 4, 6 sind zur Stirnseite 2a des Sensor 2 hin ausgerichtet, sodass die Stirnseiten der Elektroden 4, 6 einen Kontakt zur Elektrolytlösung 51 aufweisen, welche vor dem Gebrauch des Sensors 2 auf die Stirnseite 2a des Sensors 2 aufgetragen wird. Als Diaphragma wird in der Elektrochemie die Trennwand zweier Halbelemente bezeichnet. In der in Figur 2 dargestellten Anordnung trennt das Diaphragma 6d die Elektrolytlösung 51 vom Innenraum der Bezugselektrode 6. Das Diaphragma 6d besteht vorteilhafterweise aus einem keramischen Werkstoff und ist flüssigkeitsdurchlässig. Der Innenableiter 6a ist ein mit Silberchlorid beschichteter Silberdraht. Das Schaftglas 6b ist ein Borosilikatglas, welches für Luft und andere Gase undurchlässig ist. In einer bevorzugten Ausgestaltung wird als Kunststoff 7 ein Werkstoff enthaltend oder bestehend aus einer gut wärmeleitenden Substanz verwendet, was insbesondere dann von Vorteil ist, wenn der Sensor 2 eine Heizung, beispielsweise eine elektrische Heizung, zur Erwärmung aufweist.

Als Elektrolyt 6c der Bezugselektrode 6 wird vorzugsweise eine Kaliumchloridlösung gewählt. Um ein Verflüchtigen und Auslaufen des Elektrolyts 6c der Bezugselektrode 6 zu verhindern ist der Elektrolyt 6c vorteilhafterweise in ein Polymer eingebettet, beispielsweise in ein Polymerisat oder ein Polyakrylat, so dass ein leitfähiges Gel entsteht. Die Bezugselektrode 6 kann sehr klein ausgestaltet sein, und einen Aussendurchmesser von weniger als 5 mm und vorzugsweise von weniger als 2 mm aufweisen.

Während der Verwendung der Vorrichtung 2 zur Messung des Partialdrucks von Kohlendioxid wird dessen Stirnseite, wie in Figur 2 dargestellt, mit einer Elektrolytlösung 51 versehen, welche von einer gasdurchlässigen, und vorzugsweise wasserundurchlässigen Membran 50 bedeckt ist. Ausserhalb der Membran 50 befindet sich das Messgut. Die Membran 50 trennt somit die Elektrolytlösung 51 vom Messgut. Eine ideale Membran 50 wäre nur CO₂-durchlässig.

Idealerweise wird die Elektrolytlösung 51 durch das Messgut nicht verändert. Als gasdurchlässige Membran 50 eignen sich Materialien wie beispielsweise Polyäthylen (PE), Polypropylen (PP)oder Polytetrafluoräthylen (PTFE).

Im Unterschied zu dem in Figur 1 dargestellten Sensor 1 weist der in Figur 2 dargestellte, erfindungsgemässe Sensor 2 kein Silberteil beziehungsweise keine Referenzelektrode aus Silber, beschichtet mit Silberchlorid auf, sodass die Kontaktfläche 2a des Sensor 2 kein Silberchlorid aufweist, welches direkt mit der Elektrolytlösung 51 in Kontakt treten könnte. Die beiden Elektroden 4, 6 sind vorzugsweise, wie in Figur 2 dargestellt, in einem Kunststoff 7 eingebettet, und durch diesen vorzugsweise auch gegenseitig gehalten.

Der in Figur 2 dargestellte, erfindungsgemässe Sensor 2 wird vorzugsweise zum Messen des CO₂-Gehaltes im Blut des menschlichen Körpers verwendet, indem der Sensor 2 auf die Haut aufgelegt wird, und derart nichtinvasiv und vorzugsweise kontinuierlich der CO₂-Gehalt im Blut gemessen wird. Dieser Sensor 2 weist vorzugsweise eine Heizung auf, um die Hautoberfläche zu erwärmen, wobei vorzugsweise ein Wärme leitender Kunststoff 7 verwendet wird, in welchem die Elektroden 4,6 angeordnet sind.

Die Innenableiter 4a, 6a können beispielsweise mit einer analogen oder einer digitalen Signalmessvorrichtung 13 verbunden sein. Der erfindungsgemässe CO₂-Sensor könnte beispielsweise in einem wie in Figur 1 dargestellten Multifunktionalsensor angeordnet sein, welcher noch weitere Sensoren umfasst, wie den in Figur 1 dargestellten Sensor zum Erfassen der arteriellen Sauerstoffsättigung des Blutes beziehungsweise den transkutanen O₂-Gehalt.

Der erfindungsgemässe CO₂-Sensor erlaubt den CO₂-Gehalt von einer Vielzahl von Messgütern zu bestimmen, indem das Messgut mit der Membran 50 in Kontakt gebracht wird. Beispielweise könnte der CO₂-Gehalt des Blutes auch ausserhalb des Körpers derart gemessen werden, indem ein Blutstrom an der Membran 50 vorbeigeleitet wird, zum Beispiel der Blutstrom einer Herz-Kreislaufmaschine, sodass der CO₂-Gehalt dieses extrakorporalen Blutstromes messbar ist. Anstelle von Blut könnte an der Membran 50 auch ein anderes Fluid, beispielsweise eine Flüssigkeit wie Bier, oder ein gasförmiger Stoff wie Luft, vorbeigeleitet werden, um den CO₂-Gehalt dieses Medium zu messen.

## Patentansprüche

1. Vorrichtung (2) zur Messung des Partialdrucks von Kohlendioxid, umfassend ein Gehäuse (3) mit einer pH-Glaselektrode (4) sowie einer Referenzelektrode (6), wobei die Referenzelektrode (6) ein mit einem Diaphragma (6d) verschlossenes Schaftglas (6b) umfasst, wobei die pH-Glaselektrode (4) und die Referenzelektrode (6) einen Innenelektrolyt (4c, 6c) aufweisen, wobei die pH-Glaselektrode (4) und die Referenzelektrode (6) Elektrodenkontaktierungen (4a, 6a) aufweisen, die das Potential der Elektroden einer Messvorrichtung (13) zuführen, und wobei die Elektrodenkontaktierung (6a) im Innern der Referenzelektrode (6) und vorzugsweise auch die Elektrodenkontaktierung (4a) der pH-Glaselektrode (4) aus einem mit Silberchlorid beschichteten Silberdraht besteht.

2. Vorrichtung nach Anspruch 1, **dadurch gekennzeichnet, dass** diese eine Kontaktfläche (2a) ohne Silber oder Silberchlorid aufweist.

3. Vorrichtung nach einem der vorhergehenden Ansprüche, **dadurch gekennzeichnet, dass** das Diaphragma (6d) aus einem keramischen Werkstoff besteht.

4. Vorrichtung nach einem der vorhergehenden Ansprüche, **dadurch gekennzeichnet, dass** der Innenelektrolyt (6c) der Referenzelektrode (6) Kaliumchlorid enthält.

5. Vorrichtung nach Anspruch 4, **dadurch gekennzeichnet, dass** der Innenelektrolyt (6c) in einem Polymer eingebettet ist.

6. Verwendung einer Vorrichtung nach einem der vorhergehenden Ansprüche zur Messung des transkutanen Kohlendioxidpartialdrucks tcpCO₂.

7. Verwendung einer Vorrichtung nach einem der Ansprüche 1 bis 5 zur Messung des Kohlendioxidpartialdruck in einem extrakorporaler Blutstrom.

8. Verwendung einer Vorrichtung nach einem der Ansprüche 1 bis 5 in einem Sensor zur Messung einer Mehrzahl physiologischer Parameter.
